# EUROPEAN PATENT APPLICATION

(11) **EP 0 998 892 A2**
(43) Date of publication of application: **10.05.2000**
(21) Application number: 99308726.1
(22) Date of filing: 03.11.1999
(51) Int. Cl.: A61F 13/15, A61F 13/56

(54) **Absorbent article with fastening means arranged thereon**

(30) Priority: 04.11.1998 JP 31301698
(71) Applicant: UNI-CHARM CORPORATION, Kawanoe-shi Ehime-ken (JP)
(72) Inventor: Fujioka, Yoshihisa, Res. & Dev. Division, Mitoyo-gun, Kanagawa-ken 769-1602 (JP); Ono, Yoshio, Res. & Dev. Division, Mitoyo-gun, Kanagawa-ken 769-1602 (JP); Mizutani, Katsumi, Res. & Dev. Division, Mitoyo-gun, Kanagawa-ken 769-1602 (JP)
(74) Representative: Parry, Christopher Stephen

(57) **Abstract**

An absorbent article to be used being fastened and fixed on an inner face of an outer wear material, comprising a laminated piece comprising a liquid-permeable top sheet for facing a wearer, a back sheet for facing the inner face of the outer wear material, and an absorbent core interposed between the top sheet and the back sheet. The absorbent core is arranged to provide terminal regions where no absorbent core is present on both ends in a lengthwise direction of the absorbent article, and fastening means for fastening and fixing the laminated piece on the outer wear material is arranged on the back sheet at least in at least one of the terminal regions. The absorbent article can be attached in a stably fitting manner with no ready emergence of tucked-down wrinkles when the absorbent article is overlaid on the inner face of an outer wear material.

## Description

The present invention relates to an absorbent article including urine-absorbing pad (or pad against urine incontinence) for use for patients afflicted with urine incontinence and for aged individuals, sanitary napkin, or the like. More particularly, the invention relates to an absorbent article fitting in a stable manner with no emergence of tucked-down wrinkles, when the absorbent article is used inside of an outer wear material, for example underwear or diaper.

Absorbent articles such as urine-absorbing pad, pad against urine incontinence and sanitary napkin are generally used after the absorbent articles are overlaid on the inner face of an outer wear material such as underwear or diaper.

Additionally, such type of absorbent articles structurally comprise an absorbent core interposed between a liquid-permeable top sheet and a liquid-impermeable back sheet and additionally includes leg gatherings with an elastic material such as rubber being arranged on both sides of the absorbent article and vertical gatherings standing up toward the skin of a wearer (user).

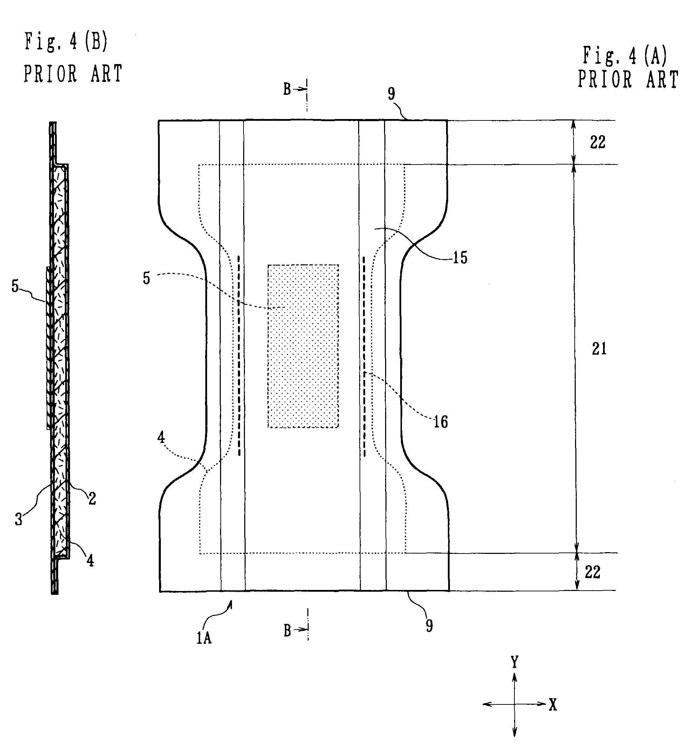

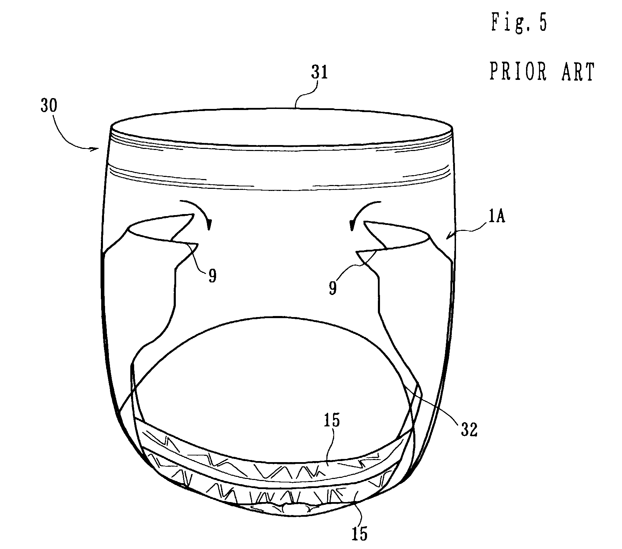

Fig. 4(A) is a plan view of the developed state of a conventional absorbent article for use being overlaid on an outer wear material, as viewed from the side of the top sheet; Fig. 4(B) is a cross-sectional view of the Fig. 4(A) along the line B-B; and Fig. 5 is a side view depicting a state of a conventional absorbent article fastened on the inner face of an underwear as an outer wear material.

As shown in the cross-sectional view of Fig. 4(B), the absorbent article 1A comprises a laminated piece composed of a liquid-permeable top sheet 2 facing toward the skin of a user, a back sheet 3 facing toward an outer wear material such as underwear and diaper, and an absorbent core 4 interposed between the top sheet 2 and the back sheet 3. Generally, such type of the absorbent article 1A includes fastening means 5 arranged on the surface of the back sheet 3, approximately on the center part of the region 21 where the absorbent core 4 is present, and the fastening means 5 functions for fastening and fixing the absorbent article 1A on an outer wear material.

As shown in Fig. 5, the absorbent article 1A is used after the fastening means 5 is fastened and fixed on the inner face of the outer wear material 30. When a user inserts legs through a waist opening 31 into leg openings 32 and then pulls up the outer wear material 30 together with the absorbent article 1A from the leg region toward the waist region until the outer wear material 30 completely fits the body, the hem 9 of the absorbent article 1A is sometimes tucked down and wrinkled toward the direction marked with arrows, because the leg or body readily contacts with or catches the hem 9 of the absorbent article 1 A. Consequently, the absorbent article shifts in position or is twisted or severely tucked down and wrinkled in the underwear, so that the absorbent article cannot receive excretes such as urine.

The invention can overcome the conventional problems described above. It is an object of the invention to provide an absorbent article which can closely fit a human body with no ready emergence of tucked-down wrinkles when the absorbent article is overlaid on the inner face of an outer wear material for use.

The above object and advantages described above can be attained by an absorbent article to be used being fastened and fixed on an inner face of an outer wear material, comprising a laminated piece comprising a liquid-permeable top sheet for facing a wearer, a back sheet for facing the inner face of the outer wear material, and an absorbent core interposed between the top sheet and the back sheet,
wherein the absorbent core is arranged to provide terminal regions where no absorbent core is present on both ends in a lengthwise direction of the absorbent article, and fastening means for fastening and fixing the laminated piece on the outer wear material is arranged on the back sheet at least in at least one of the terminal regions.

Embodiments of the invention are described below with reference to the accompanying drawings, in which:
Fig. 1(A) is a plan view of the developed state of a urine-absorbing pad (or pad against urine incontinence) for adults as one example of the inventive absorbent article, which is viewed from the side of the top sheet; and Fig. 1(B) is a cross-sectional view of the Fig. 1(A) along the line B-B;
Fig. 2 is a side view of the inventive absorbent article fastened and fixed on the inner face of an underwear as an outer wear material;
Fig. 3 is a plan view of the other embodiment of the invention;
Fig. 4(A) is a plan view of a conventional absorbent article at the developed state; and Fig. 4(B) is a cross-sectional view of the conventional absorbent article along the line B-B of Fig. 4(A); and
Fig. 5 is a side view depicting a state of a conventional absorbent article fastened and fixed on the inner face of an underwear as an outer wear material.

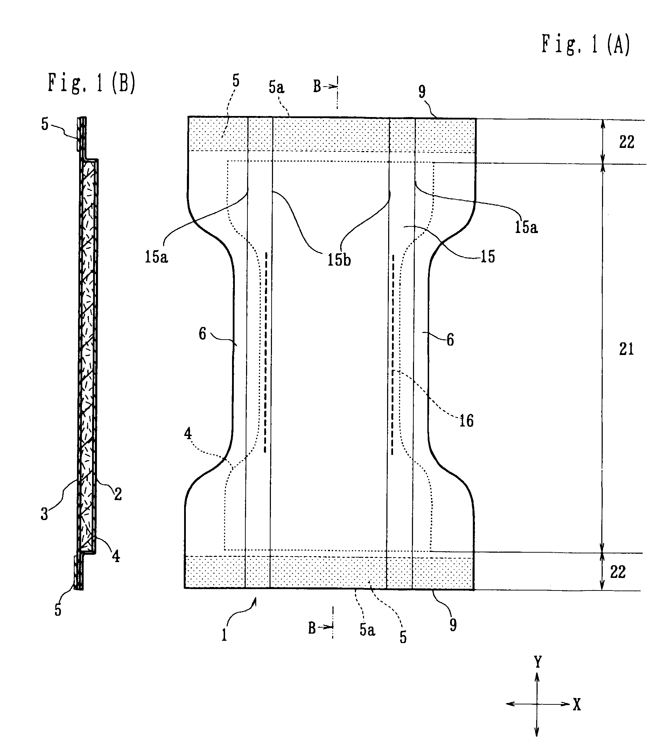

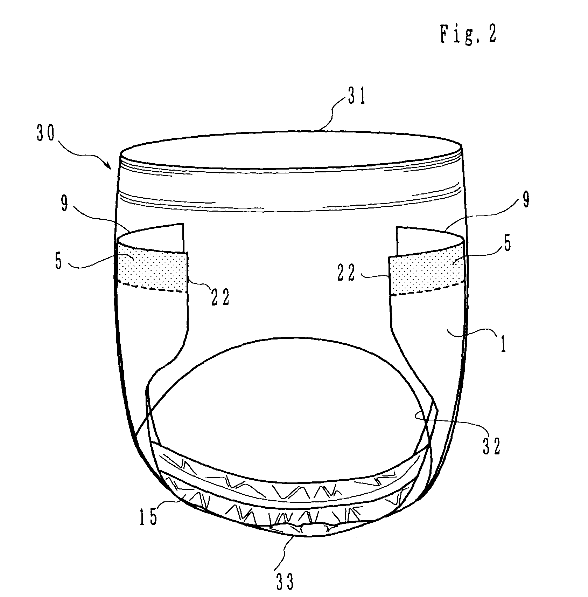

Fig. 1(A) is a plan view of a urine-absorbing pad (or pad against urine incontinence) for adults at its developed state, as one example of the inventive absorbent article, which is viewed from the side of the top sheet; Fig. 1(B) is a cross-sectional view of the Fig. 1(A) along the line B-B; and Fig. 2 is a side view of the inventive absorbent article fastened and fixed on the inner face of an underwear as an outer wear material.

As shown in the cross-sectional view of Fig. 1(B), the absorbent article 1 in Fig. 1(A) comprises a laminated piece composed of a liquid-permeable top sheet 2 facing toward the skin of a user, a back sheet 3 facing toward an outer wear material such as underwear and diaper, and an absorbent core 4 interposed between the top sheet 2 and the back sheet 3.

The back sheet 3 can be a liquid-impermeable sheet. When the back sheet 3 is to be arranged on the inner face of a diaper of pants type or open type as an outer wear material, however, a part of or the whole back sheet 3 may be prepared as being liquid permeable to transfer urine not absorbed in the absorbent core 4 of the absorbent article 1 to the absorbent material of the diaper. The liquid-impermeable back sheet 3 is prepared by using an olefinic resin sheet, a non-woven fabric, a resin sheet laminated on a non-woven fabric, or the like.

The liquid-permeable top sheet 2 is prepared by using hydrophilic fibers or hydrophilized hydrophobic fibers, and includes a non-woven fabric manufactured by point bond method, air through method, spun bond method, spun lace method or the like. The "hydrophilized hydrophobic fibers" as used herein means hydrophobic fibers subjected to hydrophilic treatment. In this hydrophilic treatment, a hydrophobic fiber is made hydrophilic by treating it with a surfactant; by chemically binding a chemical substance such as a monomer or a polymer having a hydrophilic group thereto; by subjecting it to plasma processing; by kneading it with a chemical substance having a hydrophilic group; or by treating its surface to have a profiled section.

The absorbent core 4 is prepared by using crushed pulp or a mixture of crushed pulp and super absorbent polymers (or highly water-absorbing polymers); and the absorbent core 4 comprises an absorbent sheet such as tissue, which wraps the crushed pulp or the mixture of crushed pulp and super absorbent polymers. The super absorbent polymer (or SAP) can be made of polyacrylic acid, sodium polyacrylate, polyacrylamide, polyacrylonitrile, polyvinyl alcohol, an additional polymer of maleic anhydride, a polyether, a condensed polymer, a polysaccharide such as starch or cellulose, a protein such as collagen, or the like. Examples of the SAPs include: a cross-linked compound of sodium polyacrylate, a graft copolymer of starch having sodium polyacrylate or a graft copolymer of cellulose having polyacrylonitrile chains.

In the absorbent article 1, the direction Y is a lengthwise direction, which extends from the lower abdominal region of a user toward the buttocks in use, while the direction X perpendicular to the direction Y is a crosswise direction. As shown in Fig. 1(A), the region where the absorbent core 4 is present is enclosed by the dotted line, and has a length marked with symbol 21. At both the ends in the lengthwise direction, there are provided the regions having a length marked with symbol 22, 22 where no absorbent core is present. These regions having the length 22, 22 are hereinafter designated terminal regions 22, 22.

As shown in Figs. 1(A) and 1(B), the top sheet 2 and the back sheet 3 are overlaid together while having the absorbent core 4 interposed therebetween. The top sheet 2 and the back sheet 3 come into contact with each other at the terminal regions 22, 22 and side regions 6, 6, and are bonded together by using a hot-melt type adhesive or the like.

The absorbent article 1 shown in Fig. 1(A) is overall in a so-called sand glass shape, in which the top sheet 2, the back sheet 3 and the absorbent core 4 are all in so-called sand glass shapes. Herein, only the absorbent core 4 may be in a rectangular shape or the entirety of the absorbent article 1 may be in a rectangular shape.

Additionally, the absorbent article 1 is provided with hydrophobic sheets 15, 15 extending lengthwise adjacent to two sides of the narrow region of the sand glass shape. The side edge 15a of each hydrophobic sheet 15 is bonded to the inner face (or the top sheet 2) of the absorbent article 1, whereas the other side edge 15b thereof free from the top sheet 2 is provided with longitudinally extending elastic member 16 (for example, rubber band) at its elongated state. As a result, as shown in Fig. 2, vertical gatherings for prevention of side leakage of urine or the like is formed on the absorbent article 1 standing up toward the skin of a user in use.

Symbol 30 in Fig. 2 represents an underwear (or diaper of pants type) or an outer wear material, wherein the absorbent article 1 is fastened and fixed, by means of fastening means arranged on the absorbent article 1, on the inner face of the outer wear material 30.

As shown in Figs. 1(A) and 1(B), fastening means 5, 5 are arranged in the terminal regions 22, 22 on the surface of the back sheet 3. The fastening means 5 each extends over the entire length of the terminal region 22 in the crosswise direction (direction X), i.e., extends continuously between two side edges of the absorbent article 1. In other words, the crosswise dimension of the fastening means 5 is approximately equal to the crosswise dimension of the absorbent article 1. By arranging the fastening means 5, 5 in the above manner, the hem 9 of the absorbent article 1 is hardly peeled off from the outer wear material 30 even if the hem 9 is by chance put in contact with a leg.

The fastening means 5, 5 are preferably arranged within the terminal regions 22, 22, i.e., within the regions with no absorbent core 4. The terminal regions 22, 22 can keep a degree of freeness against deformation, because no absorbent core 4 is present in the terminal regions 22, 22. Accordingly, the terminal regions 22, 22 readily follow the deformation of the outer wear material 30 such as underwear, so that the fastening means 5, 5 are more securely fastened and fixed on the inner face of the outer wear material 30 and are thus hardly detached. Herein, the fastening means 5, 5 may partially extend into the region 21, where the absorbent core 4 is present.

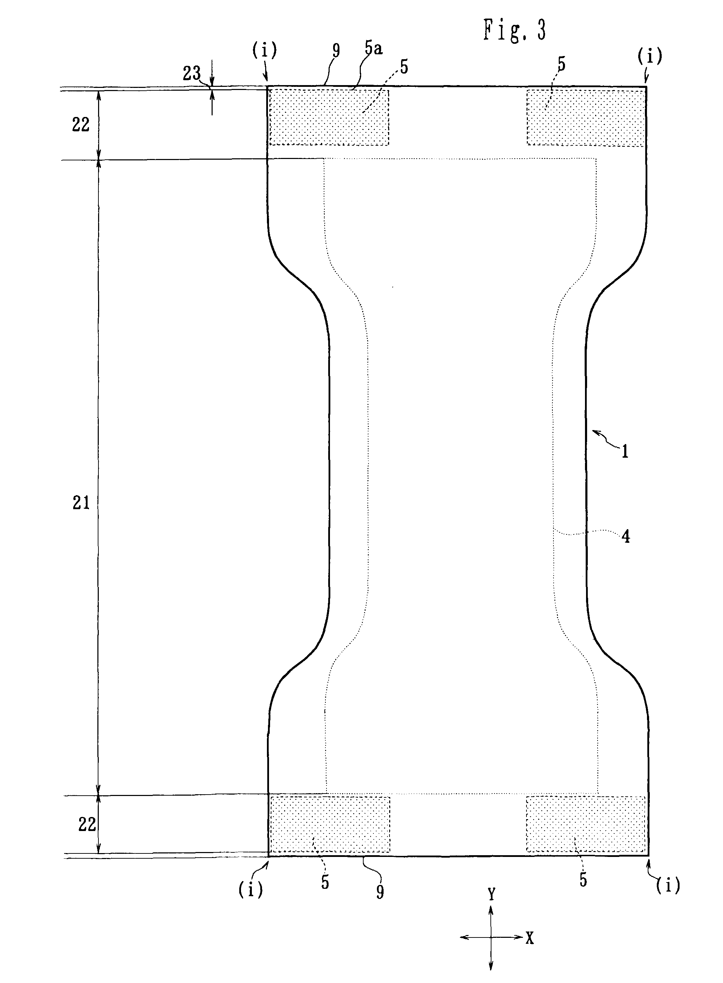

Fig. 3 is a plan view of the other embodiment of the invention. Here, only the arrangement of the fastening means 5, 5 is different from the first embodiment. Therefore, the detailed description of the portions having the same constructions as those of the first embodiment will be omitted by designating them by the common reference numerals. The hydrophobic sheet 15, 15 for forming the gatherings are omitted in Fig. 3.

In this embodiment, the fastening means 5, 5 are separately arranged on both the crosswise sides (along direction X) of the terminal regions 22, 22. For partial arrangement of the fastening means 5, 5 in the terminal regions 22, 22, preferably, the fastening means 5, 5 are separately arranged at least on both the crosswise sides of the terminal regions 22, 22, as shown in Fig. 3. The fastening means arranged on both the sides can fasten the corner (i) of the absorbent article 1 to the inner face of the outer wear material 30, whereby it is prevented for the absorbent article 1 to be peeled off from the comer (i).

Herein, the fastening means 5, 5 may satisfactorily be arranged only at the center part along the crosswise direction. Alternatively, the fastening means 5, 5 may satisfactorily be arranged separately at the center part and both the sides.

Preferably, additionally, the hems 5a, 5a of the fastening means 5 are approximately aligned with the hems 9, 9 of the absorbent article 1. If any wide distance is present between the hems 5a, 5a and the hems 9, 9, the hems 9, 9 bend inwardly when absorbent article 1 is attached on the inner face of the outer wear material 30. To prevent such inward bending, the distance 23 (see Fig. 3) between the hems 5a, 5a and the hems 9,9 is preferably within a range of 0 to 5 mm, more preferably 0 to 3 mm.

When the absorbent article 1 is attached on the inner face of the outer wear material 30, the terminal regions 22, 22 of the absorbent article 1 are fastened and fixed on the inner face of the outer wear material 30 by means of the fastening means 5, 5. Accordingly, when a user inserts the legs through the waist opening 31 of the outer wear material 30 into the leg openings 32 and subsequently pulls up the outer wear material 30 together with the absorbent article 1 from the leg region toward the waist to wear the absorbent article 1 or until the outer wear material 30 completely fits the body, the absorbent article 1 never bends inwardly even if the legs or the body contacts with the hem 9 of the absorbent article 1. Therefore, no positional shift, twisting or severe bending of the absorbent article 1 occurs in the outer wear material 30, so that the absorbent article 1 can securely receive excretes such as urine.

Any fastening means 5 capable of fastening and fixing the absorbent article 1 on the outer wear material 30 may satisfactorily be used. For example, hooking fasteners can be used. In this case, hooking fasteners with numerous protrusions with a hooking head for example in a hook shape or a mushroom shape are arranged as the fastening means 5 on the absorbent article 1, to work to fasten and fix the absorbent article 1 on a top sheet (or inner sheet) of an outer wear material, the top sheet being made of for example a non-woven fabric. Additionally, the fastening means 5 may satisfactorily be an adhesive material such as hot-melt type adhesive, rubber elastomer or the like. In this case, the absorbent article 1 can be fastened and fixed on an outer wear material, even when the top sheet of the outer wear material is prepared by using woven fabrics or sheets, other than non-woven fabrics.

So as to further prevent the positional shift of the absorbent article 1 of the invention during the wear, another fastening means may satisfactorily be arranged in the region 21, for example at the center part of the absorbent core 4 or therearound, other than the terminal regions 22.

The absorbent article 1 of the present invention should not be limited to be used with an underwear or a diaper of pants type, but may be used with an open-type diaper. Moreover, the absorbent article 1 may be used as sanitary napkin.

Furthermore, the fastening means of the invention may be arranged only in one of the terminal regions, depending on the shape and use of the absorbent article.

As has been described above, the absorbent article of the invention can be arranged securely at an appropriate position while it can be prevented for the absorbent article to be twisted or tucked down and wrinkled when it is worn.

In the foregoing specification, the invention has been described in relation to preferred embodiments and many details have been set forth for the purpose of illustration. It will be apparent to those skilled in the art that the invention is susceptible to additional embodiments and that certain of the details described herein can be varied considerably without departing from the basic principles of the invention.

Further, 'comprises/comprising' when used in this specification is taken to specify the presence of stated features, integers, steps or components but does not preclude the presence or addition of one or more other features, integers, steps, components or groups thereof.

## Claims

1. An absorbent article to be used being fastened and fixed on an inner face of an outer wear material, comprising a laminated piece comprising a liquid-permeable top sheet for facing a wearer, a back sheet for facing the inner face of the outer wear material, and an absorbent core interposed between the top sheet and the back sheet,
wherein the absorbent core is arranged to provide terminal regions where no absorbent core is present on both ends in a lengthwise direction of the absorbent article, and fastening means for fastening and fixing the laminated piece on the outer wear material is arranged on the back sheet at least in at least one of the terminal regions.

2. An absorbent article according to claim 1, wherein the fastening means is arranged within the terminal regions.

3. An absorbent article according to claim 1, wherein the fastening means is arranged to extend continuously between side edges of the terminal regions in a crosswise direction perpendicular to the lengthwise direction.

4. An absorbent article according to claim 2, wherein the fastening means is arranged to extend continuously between side edges of the terminal regions in a crosswise direction perpendicular to the lengthwise direction.

5. An absorbent article according to claim 1, wherein the distance between a hem of the terminal region of the laminated piece and a corresponding hem of the fastening means is 0 to 5 mm.

6. An absorbent article according to claim 2, wherein the distance between a hem of the terminal region of the laminated piece and a corresponding hem of the fastening means is 0 to 5 mm.

7. An absorbent article according to claim 3, wherein the distance between a hem of the terminal region of the laminated piece and a corresponding hem of the fastening means is 0 to 5 mm.

8. An absorbent article according to claim 4, wherein the distance between a hem of the terminal region of the laminated piece and a corresponding hem of the fastening means is 0 to 5 mm.

9. An absorbent article according to claim 1, wherein the fastening means is a hooking fastener.

10. An absorbent article according to claim 1, wherein the fastening means is an adhesive.
